(19)

Europäisches
Patentamt
European
Patent Office
Office européen
des brevets

(11) **EP 4 174 762 A1**

(12) # EUROPEAN PATENT APPLICATION
published in accordance with Art. 153(4) EPC

(43) Date of publication:
**03.05.2023 Bulletin 2023/18**

(21) Application number: **20943564.3**

(22) Date of filing: **30.11.2020**

(51) International Patent Classification (IPC):
***G06T 7/00*** *(2017.01)*

(52) Cooperative Patent Classification (CPC):
**G06N 20/00; G06T 7/00**

(86) International application number:
**PCT/CN2020/132798**

(87) International publication number:
**WO 2022/000977 (06.01.2022 Gazette 2022/01)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **29.06.2020 CN 202010606964
29.06.2020 CN 202010606963**

(71) Applicant: **Suzhou Rainmed Medical Technology
Co., Ltd.
Suzhou, Jiangsu 215000 (CN)**

(72) Inventors:
• **FENG, Liang**
  **Suzhou, Jiangsu 215000 (CN)**
• **LIU, Guangzhi**
  **Suzhou, Jiangsu 215000 (CN)**
• **WANG, Zhiyuan**
  **Suzhou, Jiangsu 215000 (CN)**

(74) Representative: **Boult Wade Tennant LLP
Salisbury Square House
8 Salisbury Square
London EC4Y 8AP (GB)**

(54) **DEEP LEARNING-BASED AORTIC IMAGE ACQUISITION SYSTEM**

(57)     The present application provides a system for acquiring image of aorta based on deep learning, comprising: a database device, a deep learning device, a data extraction device and an aorta acquisition device; the database device is configured for generating a database of slices of an aorta layer and a database of slices of a non-aorta layer; the deep learning device is connected to the database device, and is configured for performing deep learning on slice data, and for analyzing feature data to obtain aorta data; the data extraction device is configured for extracting feature data of CT sequence images to be processed; the aorta acquisition device is connected to the data extraction device and the deep learning device, and is configured for acquiring an image of aorta from the CT sequence images based on the deep learning model and feature data. In the present application, the deep learning model is acquired based on the feature data and the database, and the image of aorta is acquired by the deep learning model. It has the advantages of good extraction effect, high robustness, and accurate calculation results, and has high promotion value in clinical practice.

FIG.1

## Description

## TECHNICAL FIELD

The present invention refers to the technical field of coronary medicine, and in particular to systems for acquiring image of aorta based on deep learning.

## BACKGROUND

Cardiovascular diseases are leading causes of death in the industrialized world. The major forms of cardiovascular diseases are caused by chronic accumulation of fatty material in the inner tissue layers of the arteries supplying the heart, brain, kidneys and lower extremities. Progressive coronary artery diseases restrict blood flow to the heart. Due to the lack of accurate information provided through current non-invasive tests, invasive catheterization procedures are required by many patients to evaluate coronary blood flow. Thus, a need exists for non-invasive methods for quantifying blood flow in human coronary arteries to evaluate the functional significance of possible coronary artery diseases. Reliable evaluation of arterial volume will therefore be important for disposition planning to address patient needs. Recent studies have demonstrated that hemodynamic characteristics, such as flow reserve fraction (FFR), are important indicators for determining the optimal disposition for patients with arterial disease. Routine evaluation of FFR uses invasive catheterization to directly measure blood flow characteristics, such as pressure and flow rate. However, these invasive measurement techniques carry risks to the patient and can result in significant costs to the health care system.

Computed tomography arteriography is a computed tomography technique used to visualize the arterial blood vessels. For this purpose, a beam of X-rays is passed from an radiation source through the area of interest in the patient's body to obtain a projection image.

The use of empirical values to acquire images of aorta in prior art suffers from many human factors, poor consistency, and slow extraction speed.

## SUMMARY

The present invention provides a system for acquiring image of aorta based on deep learning, to solve the problems of the prior art of using empirical values to acquire images of aorta with many human factors, poor consistency and slow extraction speed.

To achieve the above, the present application provides a system for acquiring image of aorta based on deep learning, comprising: a database device, a deep learning device, a data extraction device and an aorta acquisition device;

the database device is configured for generating a database of slices of an aorta layer and a database of slices of a non-aorta layer;

the deep learning device is connected to the database device, and is configured for performing deep learning on slice data of the aorta layer and slice data of the non-aorta layer, to acquire a deep learning model, and for analyzing feature data by the deep learning model, to obtain aorta data;

the data extraction device is configured for extracting the feature data of three-dimensional data of CT sequence images or the CT sequence images to be processed;

the aorta acquisition device is connected to the data extraction device and the deep learning device, and is configured for acquiring an image of aorta from the CT sequence images based on the deep learning model and the feature data.

Optionally, the above system for acquiring image of aorta based on deep learning further comprises: a CT storage device connected to the database device and the data extraction device, configured for acquiring three-dimensional data of the CT sequence images.

Optionally, in the above system for acquiring image of aorta based on deep learning, the database device comprises: an image processing structure, a slice data storage structure for aorta layer and a slice data storage structure for non-aorta layer, wherein the slice data storage structure for aorta layer, the slice data storage structure for non-aorta layer and the CT storage device are all connected to the image processing structure;

the image processing structure is configured for removing new images of the lung, descending aorta, spine and ribs from the CT sequence images;

the slice data storage structure for aorta layer is configured for acquiring slice data of the aorta layer from the new images; and

the slice data storage structure for non-aorta layer is configured for acquiring the remaining slice data from the new images with the slices within the slice data storage structure for aorta layer removed, i.e., the slice data of non-aorta layer.

Optionally, in the above system for acquiring image of aorta based on deep learning, the image processing structure comprises: a grayscale histogram unit, a grayscale volume acquisition unit, a lung tissue removal unit, an extraction unit for gravity center of heart, an extraction unit for gravity center of spine, an extraction unit for image of descending aorta, and a new image acquisition unit;

the grayscale histogram unit is connected to the CT storage unit, and is configured for plotting a grayscale histogram of each group of CT sequence images;

the grayscale volume acquisition unit is connected to the grayscale histogram structure, and is configured for, along a direction of the end point M to the original point O of the grayscale histogram, acquiring a volume of each grayscale value region from point M to point M-1, from point M to point M-2 successively, until from point M to point O; acquiring a volume ratio V of the volume of each grayscale value region to a volume of the total region from point M to point O;

the lung tissue removal unit is connected to the grayscale volume acquisition unit, and is configured for setting a lung grayscale threshold $Q_{lung}$ based on medical knowledge and CT imaging principle, if a grayscale value in the grayscale histogram being less than $Q_{lung}$, removing an image corresponding to the grayscale value to obtain a first image with the lung tissue removed;

the extraction unit for gravity center of heart is connected to the grayscale volume acquisition unit, and is configured for acquiring a gravity center of heart $P_2$, if V = b, picking a start point corresponding to the grayscale value region, projecting the start point onto the first image, acquiring a three-dimensional image of a heart region, and picking a physical gravity center of the three-dimensional image of the heart region $P_2$, wherein b denotes a constant, 0.2 < b < 1.

The extraction unit for gravity center of spine is connected to the CT storage device and the extraction unit for gravity center of heart, and is configured for acquiring a gravity center of spine Pi, if V = a, picking a start point corresponding to a grayscale value region, projecting the start point onto the CT three-dimensional image, acquiring a three-dimensional image of a bone region, and picking a physical gravity center of the three-dimensional image of the bone region Pi, wherein a denotes a constant, 0 < a < 0.2.

The extraction unit for image of descending aorta is connected to the extraction structure for gravity center of heart, the extraction structure for gravity center of spine and the lung tissue removal unit, and is configured for acquiring an image of descending aorta of each group of CT sequence images based on the gravity center of heart and the gravity center of spine;

the new image acquisition unit is connected to the extraction unit for image of descending aorta, the lung tissue removal unit, the slice data storage structure for aorta layer and the slice data storage structure for non-aorta layer, and is configured for removing the lung, descending aorta, spine and ribs from CT sequence images, to acquire new images.

Optionally, in the above system for acquiring image of aorta based on deep learning, the region delineation unit for descending aorta comprises: an average grayscale value acquisition module, a layered slice module and a binarization processing module;

the average grayscale value acquisition module is connected to the lung tissue removal unit and the grayscale histogram structure, and is configured for acquiring one or more pixel points PO within the first image with a grayscale value greater than the grayscale threshold for the descending aorta $Q_{descending}$, and calculating an average grayscale value $\overline{Q}_1$ of the one or more pixel points PO;
the layered slice module is connected to the average grayscale value acquisition module and the lung tissue removal unit, and is configured for layered slicing the first image starting from its bottom layer to obtain a first group of two-dimensional sliced images;
the binarization processing module is connected to the layered slice module and the grayscale histogram structure, and is configured for, based on

$$\begin{cases} Q_k < Q_{descending}, P(k) = 0 \\ Q_{descending} \le Q_k \le 2\overline{Q}_1, P(k) = 1 \\ Q_k > 2\overline{Q}_1, P(k) = 0 \end{cases}$$

, binarizing the sliced image, removing impurity points from the first image to obtain a binarized image, wherein k is a positive integer, $Q_k$ denotes the grayscale value corresponding to the k-th pixel point PO, and P(k) denotes the pixel value corresponding to the k-th pixel point PO.

Optionally, in the above system for acquiring image of aorta based on deep learning, the region delineation unit for descending aorta further comprises: an rough acquisition module and an accurate acquisition module;

the rough acquisition module is connected to the binarization processing module, and is configured for setting an radius threshold of a circle formed from the descending aorta to an edge of the heart to $r_{threshold}$, acquiring an approximate region of the spine and an approximate region of the descending aorta based on the distance between the descending aorta and the heart being less than the distance between the spine and the heart;
the accurate acquisition module is connected to the rough acquisition module, and is configured for removing one or more error pixel points based on the approximate region of the descending aorta, i.e., a circle corresponding to the descending aorta.

Optionally, in the above system for acquiring image of aorta based on deep learning, the data extraction device comprises: a connected domain structure and a feature data acquisition structure;

the connected domain structure is connected to the new image acquisition unit and is configured for acquiring a plurality of binarized images of the CT sequence images to be processed from the new image acquisition unit;

the feature data acquisition structure is connected to the connected domain structure, and is configured for acquiring a connected domain of each binarized image successively starting from the top layer, as well as a proposed circle center $C_k$, an area $S_k$, a proposed circle radius $R_k$, and a distance $C_k$-$C_{(k-1)}$ between the circle centers of two adjacent layers, a distance $C_k$-$C_1$ from the circle center $C_k$ of each layer of slice to the circle center of the top layer Ci, and an area $M_k$ of all pixels whose pixel points are greater than 0 in a layer pixel and whose pixel points are equal to 0 in the previous layer pixel and a filtered area $H_k$ corresponding to the connected domain, wherein k denotes the k-th layer of slice, $k \geq 1$; i.e., the feature data.

Optionally, in the above system for acquiring image of aorta based on deep learning, the feature data acquisition structure is provided with a data processing unit, as well as a circle center acquisition unit, an area acquisition unit and an radius acquisition unit, respectively, connected to the data processing unit;

the data processing unit is configured for detecting 3 layers of slice successively starting from the top layer by using the Hoff detection algorithm, and obtaining 1 circle center and 1 radius from each layer of slice, forming 3 circles respectively; removing points with larger deviations from 3 circle centers to obtain a seed point Pi of the descending aorta; acquiring a connected domain $A_1$ of the layer where the seed point Pi is located; acquiring a gravity center of the connected domain $A_1$ as the proposed circle center Ci, and acquiring the area $S_1$ of the connected domain $A_1$ and the proposed circle radius Ri; acquiring a connected domain $A_2$ of the layer where the seed point Pi is located, by using the Ci as a seed point; expanding the connected domain $A_1$ to obtain an expanded region Di, removing a portion overlapping with the expanded region Di from the connected domain $A_2$ to obtain a connected domain $A_2$'; setting a volume threshold $V_{threshold}$ for the connected domain, if a volume $V_2$ of the connected domain $A_2$' being less than $V_{threshold}$, removing one or more points that are too far from the circle center $C_1$ of the previous layer, acquiring the filtered area $H_k$, making the gravity center of the connected domain $A_2$' as a proposed circle center $C_2$, acquiring an area $S_2$ of the connected domain $A_2$ and a proposed circle ra-

dius $R_2$; repeating the method of the connected domain $A_2$, acquiring a connected domain of each binarized image successively, as well as a proposed circle center $C_k$, an area $S_k$, a proposed circle radius $R_k$, and a distance $C_k$-$Q_{(k-1)}$ between the circle centers of two adjacent layers, a distance $C_k$-$C_1$ from the circle center $C_k$ of each layer of slice to the circle center of the top layer Ci corresponding to the connected domain;

the circle center acquisition unit is configured for storing the proposed circle centers Ci, $C_2$... $C_k$ ...;

the area acquisition unit is configured for storing the areas Si, $S_2$ ... $S_k$ ..., and the filtered areas Hi, $H_2$ ... $H_k$ ...;

the radius acquisition unit is configured for storing the proposed circle radii $R_1$, $R_2$ ... $R_k$ ... .

Optionally, in the above system for acquiring image of aorta based on deep learning, the aorta acquisition device comprises: a gradient edge structure and an acquisition structure for image of aorta;

the gradient edge structure is connected to the deep learning device and is configured for expanding aorta data; multiplying the expanded aorta data with original CT sequence image data, and calculating a gradient of each pixel point to obtain gradient data; extracting a gradient edge based on the gradient data; subtracting the gradient edge from the expanded aorta data;

the acquisition structure for image of aorta is connected to the new image acquisition unit and the gradient edge structure, and is configured for generating a list of seed points based on a proposed circle center; extracting a connected domain based on the list of seed points, to obtain an image of aorta.

The beneficial effects resulting from the solutions provided by embodiments of the present application include at least that:
the present application provides a system for acquiring image of aorta based on deep learning, wherein a deep learning model is acquired based on feature data and database, and an image of aorta is acquired by the deep learning model. It has the advantages of good extraction effect, high robustness, and accurate calculation results, and has high promotion value in clinical practice.

## BRIEF DESCRIPTION OF DRAWINGS

The drawings illustrated herein are used to provide a further understanding of the present invention, form a part of the present invention, and the schematic embodiments of the invention and their descriptions are used to explain the present invention and do not constitute an undue limitation of the present invention. Wherein:

FIG. 1 is a structure block diagram of an embodiment of the system for acquiring image of aorta based on deep learning of the present application;

FIG. 2 is a structure block diagram of another embodiment of the system for acquiring image of aorta based on deep learning of the present application;

FIG. 3 is a structure block diagram of a database device 100 of the present application;

FIG. 4 is a structure block diagram of an image processing structure 110 of the present application;

FIG. 5 is a structure block diagram of an image storage structure for descending aorta 160 of the present application;

FIG. 6 is a structure block diagram of an region delineation unit for descending aorta 162 of the present application;

FIG. 7 is a structure block diagram of a data extraction device 300 of the present application;

FIG. 8 is a structure block diagram of an aorta acquisition device 400 of the present application.

## DETAILED DESCRIPTION

In order to make the purpose, technical solutions and advantages of the present invention clearer, the following will be a clear and complete description of the technical solutions of the present invention in conjunction with specific embodiments of the present invention and the corresponding drawings. Obviously, the described embodiments are only a part of the embodiments of the present invention, and not all of them. Based on the embodiments in the present invention, all other embodiments obtained by a person of ordinary skill in the art without making creative labor fall in the protection scope of the present invention.

A number of embodiments of the present invention will be disclosed in the following figures, and for the sake of clarity, many of the practical details will be described together in the following description. It should be understood, however, that these practical details should not be used to limit the present invention. That is, in some embodiments of the present invention, these practical details are not necessary. In addition, for the sake of simplicity, some of the commonly known structures and components will be illustrated in the drawings in a simple schematic manner.

The use of empirical values to acquire images of aorta in prior art suffers from many human factors, poor consistency, and slow extraction speed.

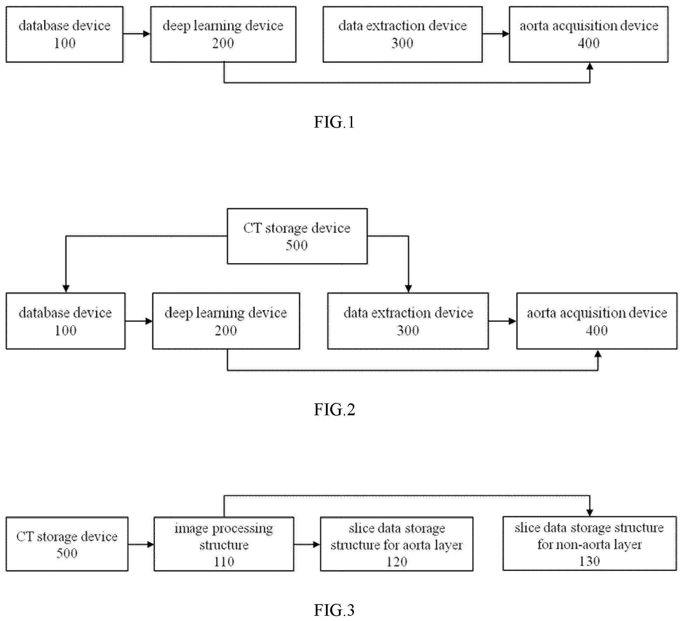

In order to solve the above problems, as shown in FIG. 1, the present application provides a system for acquiring image of aorta based on deep learning, comprising: a database device 100, a deep learning device 200, a data extraction device 300 and an aorta acquisition device 400; the database device 100 is configured for generating a database of slices of an aorta layer and a database of slices of a non-aorta layer; the deep learning device 200 is connected to the database device 100, and is configured for performing deep learning on slice data of the aorta layer and slice data of the non-aorta layer, to acquire a deep learning model, and for analyzing feature data by the deep learning model, to obtain aorta data; the data extraction device 300 is configured for extracting feature data of three-dimensional data of CT sequence images or CT sequence images to be processed; the aorta acquisition device is connected to the data extraction device 300 and the deep learning device 200, and is configured for acquiring an image of aorta from the CT sequence images based on the deep learning model and feature data.

As shown in FIG. 2, an embodiment of the present application further comprises: a CT storage device 500 connected to the database device 100 and the data extraction device 300, for acquiring three-dimensional data of the CT sequence images.

As shown in FIG. 3, in an embodiment of the present application, the database device 100 comprises: an image processing structure 110, a slice data storage structure for aorta layer 120 and a slice data storage structure for non-aorta layer 130, where the slice data storage structure for aorta layer 110, the slice data storage structure for non-aorta layer 120 and the CT storage device 500 are all connected to the image processing structure 110; the image processing structure 110 is configured for removing new images of the lung, descending aorta, spine and ribs from the CT sequence images; the slice data storage structure for aorta layer 120 is configured for acquiring slice data of the aorta layer from the new images; and the slice data storage structure for non-aorta layer 130 is configured for acquiring the remaining slice data from the new images with the slices within the slice data storage structure for aorta layer 120 removed, i.e., the slice data of non-aorta layer.

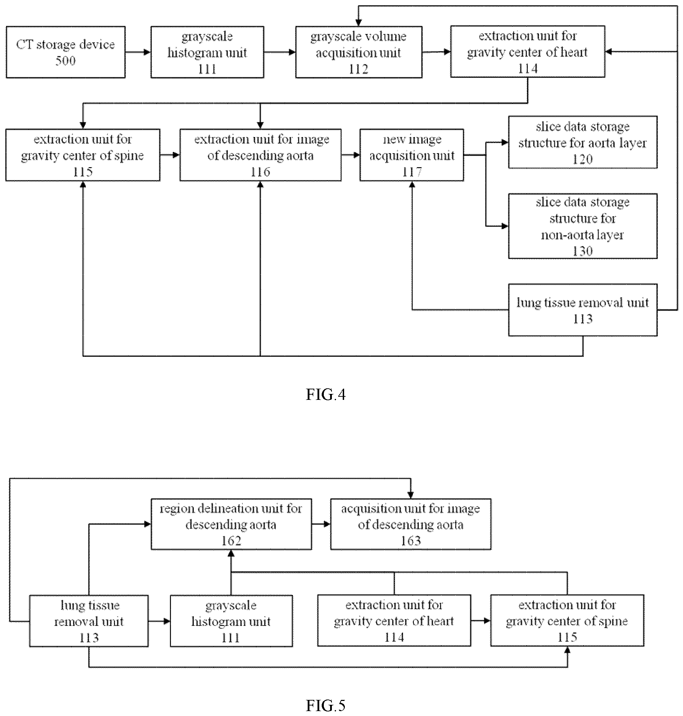

As shown in FIG. 4, in one embodiment of the present application, the image processing structure 110 comprises: a grayscale histogram unit 111, a grayscale volume acquisition unit 112, a lung tissue removal unit 113, an extraction unit for gravity center of heart 114, an extraction unit for gravity center of spine 115, an extraction unit for image of descending aorta 116, and a new image acquisition unit 117; the grayscale histogram unit 111 is connected to the CT storage unit 500, and is configured for plotting a grayscale histogram of each group of CT sequence images; the grayscale volume acquisition unit 112 is connected to the grayscale histogram unit 111, and is configured for, along a direction of the end point M to the original point O of the grayscale histogram, acquiring a volume of each grayscale value region from point M to point M-1, from point M to point M-2 successively, until from point M to point O; acquiring a volume ratio V of the volume of each grayscale value region to a volume of the total region from point M to point O; the lung tissue removal unit 113 is connected to the grayscale volume acquisition unit 112, and is configured for setting a lung grayscale threshold $Q_{lung}$ based on medical knowledge and CT imaging principle, if a grayscale value in the grayscale histogram 111 being less than $Q_{lung}$, removing an image corresponding to the grayscale value to obtain a first image with the lung tissue removed; the extraction unit for gravity center of heart 114 is connected to the grayscale volume acquisition unit 112 and the lung tissue removal unit 113, and is configured for acquiring a gravity center of heart $P_2$, if V = b, picking a start point corresponding to the grayscale value region, projecting the start point onto the first image, acquiring a three-dimensional image of a heart region, and picking a physical gravity center of the three-dimensional image of the heart region $P_2$, wherein b denotes a constant, 0.2 < b < 1. The extraction unit for gravity center of spine 115 is connected to the lung tissue removal unit 113 and the extraction unit for gravity center of heart 114, and is configured for acquiring a gravity center of spine Pi, if V = a, picking a start point corresponding to a grayscale value region, projecting the start point onto the CT three-dimensional image, acquiring a three-dimensional image of a bone region, and picking a physical gravity center of the three-dimensional image of the bone region Pi, wherein a denotes a constant, 0 < a < 0.2. The extraction unit for image of descending aorta 116 is connected to the extraction unit for gravity center of heart 114, the extraction unit for gravity center of spine 115 and the lung tissue removal unit 113, and is configured for acquiring an image of descending aorta of each group of CT sequence images based on the gravity center of heart and the gravity center of spine; the new image acquisition unit 117 is connected to the extraction unit for image of descending aorta 116, the lung tissue removal unit 113, the slice data storage structure for aorta layer 120 and the slice data storage structure for non-aorta layer 130, and is configured for removing the lung, descending aorta, spine and ribs from CT sequence images, to acquire new images.

In the present application, by first screening out the center of gravity for the heart and the spine, locating the position of the heart and the spine, and then acquiring the image of the descending aorta based on the position of the heart and the spine, computation burden is reduced, with simple algorithms, easy operation, fast computing speed, scientific design and accurate image processing.

As shown in FIG. 5, in an embodiment of the present application, the extraction unit for image of descending aorta 116 comprises: an region delineation unit for descending aorta 162, an acquisition unit for image of descending aorta 163; the region delineation unit for descending aorta 162 is connected to the grayscale histogram unit 111, the extraction unit for gravity center of heart 114, the extraction unit for gravity center of spine 115 and the lung tissue removal unit 113, and is configured for projecting the gravity center of heart $P_2$ onto the first image to obtain a circle center of the heart Oi; setting a grayscale threshold for the descending aorta $Q_{descending}$, and binarizing the first image; acquiring a circle corresponding to the descending aorta based on a distance from the descending aorta to the circle center of the heart Oi and a distance from the spine to the circle center of the heart Oi; the acquisition unit for image of descending aorta 163 is connected to the lung tissue removal unit 113 and the region delineation unit for descending aorta 162, and is configured for acquiring an image of descending aorta from the CT sequence images.

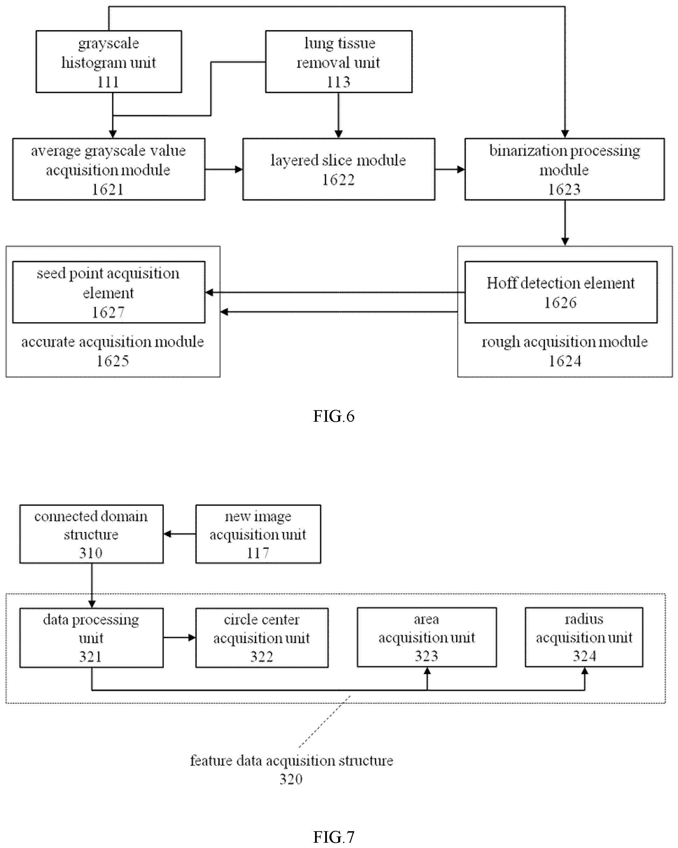

As shown in FIG. 6, in an embodiment of the present application, the region delineation unit for descending aorta 162 comprises: an average grayscale value acquisition module 1621, a layered slice module 1622 and a binarization processing module 1623; the average grayscale value acquisition module 1621 is connected to the lung tissue removal unit 113 and the grayscale histogram unit 111, and is configured for acquiring one or more pixel points PO within the first image with a grayscale value greater than the grayscale threshold for the descending aorta $Q_{descending}$, and calculating an average grayscale value $\overline{Q}_1$ of the one or more pixel points PO; the layered slice module 1622 is connected to the average grayscale value acquisition module 1621 and the lung tissue removal unit 113, and is configured for layered slicing the first image starting from its bottom layer to obtain a first group of two-dimensional sliced images; the binarization processing module 1623 is connected to the layered slice module 1622 and the grayscale histogram unit 111, and is configured for, based on

$$\left\{ \begin{array}{l} Q_k < Q_{descending}, P(k) = 0 \\ Q_{descending} \le Q_k \le 2\overline{Q}_1, \ P(k) = 1 \\ Q_k > 2\overline{Q}_1, P(k) = 0 \end{array} \right\}$$

, binarizing the sliced image, removing impurity points from the first image to obtain a binarized image, wherein k is a positive integer, $Q_k$ denotes the grayscale value corresponding to the k-th pixel point PO, and P(k) denotes the pixel value corresponding to the k-th pixel point PO.

As shown in FIG. 6, in an embodiment of the present application, the region delineation unit for descending aorta 162 further comprises: an rough acquisition module 1624 and an accurate acquisition module 1625; the rough acquisition module 1624 is connected to the binarization processing module 1623, and is configured for setting an radius threshold of a circle formed from the descending aorta to an edge of the heart to $r_{threshold}$, acquiring an approximate region of the spine and an approximate region of the descending aorta based on the distance between the descending aorta and the heart being less than the distance between the spine and the heart; the accurate acquisition module 1625 is connected to the rough acquisition module 1624, and is configured for removing one or more error pixel points based on the approximate region of the descending aorta, i.e., a circle corresponding to the descending aorta. A Hoff detection element 1626 is provided in the rough acquisition module 1624; the Hoff detection element 1626 is configured for determining an approximate region of the descending aorta based on the following principles: if a circle obtained by the Hoff detection algorithm meets the condition that its radius $r > r_{threshold}$, then this circle is the circle corresponding to the spine and is the approximate region of the spine, and the center and radius need not to be recorded; if a circle obtained by the Hoff detection algorithm meets the condition that its radius $r \leq r_{threshold}$, then this circle may be the circle corresponding to the descending aorta and is the approximate region of the descending aorta, and the center and radius need to be recorded. A seed point acquisition element 1627 is provided in the accurate acquisition module 1625; the seed point acquisition element 1627 is connected to the Hoff detection element 1626, and is configured for screening the centers and radii of the circles within the approximate region of the descending aorta, removing the circles with centers of large deviations between adjacent slices, i.e., removing the one or more error pixel points, and forming a list of seed points of the descending aorta.

As shown in FIG. 7, in an embodiment of the present application, the data extraction device 300 comprises: a connected domain structure 310 and a feature data acquisition structure 320; the connected domain structure 310 is connected to the new image acquisition unit 117 and is configured for acquiring a plurality of binarized images of the CT sequence images to be processed from the new image acquisition unit 117; the feature data acquisition structure 320 is connected to the connected domain structure 310 and is configured for acquiring a connected domain of each binarized image successively starting from the top layer, as well as a proposed circle center $C_k$, an area $S_k$, a proposed circle radius $R_k$, and a distance $C_k$-$C_{(k-1)}$ between the circle centers of two adjacent layers, a distance $C_k$-$C_1$ from the circle center $C_k$ of each layer of slice to the circle center of the top layer Ci, and an area $M_k$ of all pixels whose pixel points are greater than 0 in a layer pixel and whose pixel points are equal to 0 in the previous layer pixel and a filtered area $H_k$ corresponding to the connected domain, wherein k denotes the k-th layer of slice, $k \geq 1$; i.e., the feature data.

As shown in FIG. 7, in an embodiment of the present application, the feature data acquisition structure 320 is provided with a data processing unit 321, and a circle center acquisition unit 322, an area acquisition unit 323 and an radius acquisition unit 324, respectively, connected to the data processing unit 321; the data processing unit 321 is configured for detecting 3 layers of slice successively starting from the top layer by using the Hoff detection algorithm, and obtaining 1 circle center and 1 radius from each layer of slice, forming 3 circles respectively; removing points with larger deviations from 3 circle centers to obtain a seed point Pi of the descending aorta; acquiring a connected domain $A_1$ of the layer where the seed point Pi is located; acquiring a gravity center of the connected domain $A_1$ as the proposed circle center Ci, and acquiring the area $S_1$ of the connected domain $A_1$ and the proposed circle radius Ri; acquiring a connected domain $A_2$ of the layer where the seed point Pi is located, by using the Ci as a seed point; expanding the connected domain $A_1$ to obtain an expanded region Di, removing a portion overlapping with the expanded region Di from the connected domain $A_2$ to obtain a connected domain $A_2'$; setting a volume threshold $V_{threshold}$ for the connected domain, if a volume $V_2$ of the connected domain $A_2'$ being less than $V_{threshold}$, removing one or more points that are too far from the circle center $C_1$ of the previous layer, acquiring the filtered area $H_k$, making the gravity center of the connected domain $A_2'$ as a proposed circle center $C_2$, acquiring an area $S_2$ of the connected domain $A_2$ and a proposed circle radius $R_2$; repeating the method of the connected domain $A_2$, acquiring a connected domain of each binarized image successively, as well as a proposed circle center $C_k$, an area $S_k$, a proposed circle radius $R_k$, and a distance $C_k$-$Q_{(k-1)}$ between the circle centers of two adjacent layers, a distance $C_k$-$C_1$ from the circle center $C_k$ of each layer of slice to the circle center of the top layer Ci corresponding to the connected domain, the circle center acquisition unit is configured for storing the proposed circle centers $C_1$, $C_2$ ... $C_k$ ...; the area acquisition unit is configured for storing the areas $S_1$, $S_2$ ... $S_k$ ..., and the filtered areas $H_1$, $H_2$ ... $H_k$ ...; the radius acquisition unit is configured for storing the proposed circle radii $R_1$, $R_2$ ... $R_k$ ....

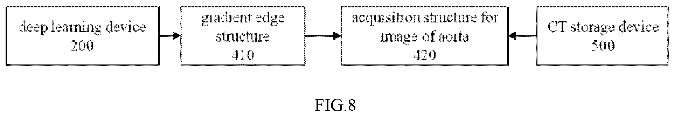

As shown in FIG. 8, in an embodiment of the present application, the aorta acquisition device 400 comprises: a gradient edge structure 410 and an acquisition structure for image of aorta 420, the gradient edge structure 410 is connected to the deep learning device 200 and is configured for expanding aorta data; multiplying the expanded aorta data with original CT sequence image data, and calculating a gradient of each pixel point to obtain gradient data; extracting a gradient edge based on the gradient data; subtracting the gradient edge from the expanded aorta data; the acquisition structure for image of aorta 420 is connected to the CT storage device 500 and the gradient edge structure 410, and is configured for generating a list of seed points based on a proposed circle center; extracting a connected domain based on the list of seed points, to obtain an image of aorta.

Those skilled in the art know that aspects of the present invention can be implemented as systems, methods, or computer program products. As such, aspects of the present invention may be implemented in the form of: a fully hardware implementation, a fully software implementation (including firmware, resident software, microcode, etc.), or a combination of hardware and software aspects, collectively referred to herein as a "circuit", "module" or "system". In addition, in some embodiments, aspects of the present invention may also be implemented in the form of a computer program product in one or more computer-readable media containing computer-readable program code. Embodiments of the methods and/or systems of the present invention may be implemented in a manner that involves performing or completing selected tasks manually, automatically, or in a combination thereof.

For example, the hardware for performing the selected tasks based on the embodiments of the present invention may be implemented as a chip or circuit. As software, the selected tasks based on the embodiments of the present invention may be implemented as a plurality of software instructions to be executed by a computer using any appropriate operating system. In exemplary embodiments of the present invention, one or more tasks, as in the exemplary embodiments based on the methods and/or systems herein, is performed by a data processor, such as a computing platform for executing a plurality of instructions. Optionally, the data processor includes volatile storage for storing instructions and/or data, and/or non-volatile storage for storing instructions and/or data, such as a magnetic hard disk and/or removable media. Optionally, a network connection is also provided. Optionally, a display and/or user input device, such as a keyboard or mouse, is also provided.

Any combination of one or more computer readable may be utilized. A computer-readable medium may be a computer-readable signal medium or a computer-readable storage medium. A computer-readable storage medium may be, for example - but not limited to - an electrical, magnetic, optical, electromagnetic, infrared, or semiconductor system, device or component, or any combination thereof. More specific examples of computer-readable storage media (a non-exhaustive list) would include each of the following:
An electrical connection having one or more wires, a portable computer disk, a hard disk, a random access memory (RAM), a read-only memory (ROM), an erasable programmable read-only memory (EPROM or flash memory), an optical fiber, a portable compact disk read-only memory (CD-ROM), an optical storage component, a magnetic storage component, or any suitable combination of the foregoing. In this specification, the computer-readable storage medium may be any tangible medium that contains or stores a program that can be used by or in combination with an instruction execution system, device or component.

The computer-readable signal medium may include a data signal propagated in a baseband or as part of a carrier wave that carries computer-readable program code. This propagated data signal can take a variety of forms, including but not limited to electromagnetic signals, optical signals or any suitable combination of the above. The computer-readable signal medium may also be any computer-readable medium other than a computer-readable storage medium that sends, propagates, or transmits a program for being used by or in conjunction with an instruction execution system, device or component.

The program code contained on the computer-readable medium may be transmitted using any suitable medium, including (but not limited to) wireless, wired, fiber optic, RF, etc., or any suitable combination of the above.

For example, computer program code for performing operations of aspects of the present invention may be written in any combination of one or more programming languages, including object-oriented programming languages such as Java, Smalltalk, C++, and conventional procedural programming languages such as "C" programming language or the like. The program code may be executed entirely on an user's computer, partially on an user's computer, as a stand-alone software package, partially on an user's computer and partially on a remote computer, or entirely on a remote computer or server. In the case of a remote computer, the remote computer may be connected to an user's computer via any kind of network - including a local area network (LAN) or a wide area network (WAN) - or, may be connected to an external computer (e.g., using an Internet service provider to connect via the Internet).

It should be understood that each block of the flowchart and/or block diagram, and a combination of respective blocks in the flowchart and/or block diagram, may be implemented by computer program instructions. These computer program instructions may be provided to a processor of a general purpose computer, a specialized computer, or other programmable data processing device, thereby producing a machine such that these computer program instructions, when executed by the processor of the computer or other programmable data processing device, produce a device that implements a function/action specified in one or more of the blocks in the flowchart and/or block diagram.

These computer program instructions may also be stored in a computer-readable medium that causes a computer, other programmable data processing device, or other apparatus to operate in a particular manner such that the instructions stored in the computer-readable medium result in an article of manufacture that includes instructions to implement the function/action specified in one or more blocks in the flowchart and/or block diagram.

Computer program instructions may also be loaded onto a computer (e.g., a coronary artery analysis system) or other programmable data processing apparatus to cause a series of operational steps to be performed on the computer, other programmable data processing apparatus or other apparatus to produce a computer-implemented process, such that the instructions executed on the computer, other programmable device or other apparatus provide a process for implementing the function/action specified in a block of the flowchart and/or one or more block diagram.

The above specific examples of the present invention further detail the purpose, technical solutions and beneficial effects of the present invention. It should be understood that the above are only specific embodiments of the present invention and are not intended to limit the present invention, and that any modifications, equivalent replacements, improvements, etc. made within the spirit and principles of the present invention shall be included within the protection scope of the present invention.

**Claims**

1. A system for acquiring image of aorta based on deep learning, **characterized by** comprising: a database device, a deep learning device, a data extraction device and an aorta acquisition device;

   the database device is configured for generating a database of slices of an aorta layer and a database of slices of a non-aorta layer;
   the deep learning device is connected to the database device, and is configured for performing deep learning on slice data of the aorta layer and slice data of the non-aorta layer, to acquire a deep learning model, and for analyzing feature data by the deep learning model, to obtain aorta data;
   the data extraction device is configured for extracting the feature data of three-dimensional data of CT sequence images or the CT sequence images to be processed;
   the aorta acquisition device is connected to the data extraction device and the deep learning device, and is configured for acquiring an image of aorta from the CT sequence images based on the deep learning model and the feature data.

2. The system for acquiring image of aorta based on deep learning according to claim 1, **characterized by** further comprising: a CT storage device connected to the database device and the data extraction device, configured for acquiring three-dimensional data of the CT sequence images.

3. The system for acquiring image of aorta based on deep learning according to claim 2, **characterized in that**, the database device comprises: an image processing structure, a slice data storage structure for aorta layer and a slice data storage structure for non-aorta layer, wherein the slice data storage structure for aorta layer, the slice data storage structure for non-aorta layer and the CT storage device are all connected to the image processing structure;

   the image processing structure is configured for removing new images of the lung, descending aorta, spine and ribs from the CT sequence images;
   the slice data storage structure for aorta layer is configured for acquiring slice data of the aorta layer from the new images; and
   the slice data storage structure for non-aorta layer is configured for acquiring the remaining slice data from the new images with the slices within the slice data storage structure for aorta layer removed, i.e., the slice data of non-aorta layer.

4. The system for acquiring image of aorta based on deep learning according to claim 3, **characterized in that**, the image processing structure comprises: a grayscale histogram unit, a grayscale volume acquisition unit, a lung tissue removal unit, an extraction unit for gravity center of heart, an extraction unit for gravity center of spine, an extraction unit for image of descending aorta, and a new image acquisition unit;

   the grayscale histogram unit is connected to the CT storage unit, and is configured for plotting a grayscale histogram of each group of CT sequence images;
   the grayscale volume acquisition unit is connected to the grayscale histogram structure, and is configured for, along a direction of the end point M to the original point O of the grayscale histogram, acquiring a volume of each grayscale value region from point M to point M-1, from point M to point M-2 successively, until from point M to point O; acquiring a volume ratio V of the volume of each grayscale value region to a volume of the total region from point M to point O;
   the lung tissue removal unit is connected to the grayscale volume acquisition unit, and is configured for setting a lung grayscale threshold $Q_{lung}$ based on medical knowledge and CT imaging principle, if a grayscale value in the grayscale histogram being less than Qlung, removing an image corresponding to the grayscale value to obtain a first image with the lung tissue removed;
   the extraction unit for gravity center of heart is connected to the grayscale volume acquisition unit and the lung tissue removal unit, and is configured for acquiring a gravity center of heart $P_2$, if V = b, picking a start point corresponding to the grayscale value region, projecting the start point onto the first image, acquiring a three-dimensional image of a heart region, and picking a physical gravity center of the three-dimensional image of the heart region $P_2$, wherein b denotes a constant, 0.2 < b < 1;
   the extraction unit for gravity center of spine is connected to the CT storage device and the extraction unit for gravity center of heart, and is configured for acquiring a gravity center of spine Pi, if V = a, picking a start point corresponding to a grayscale value region, projecting the start point onto the CT three-dimensional image, acquiring a three-dimensional image of a bone region, and picking a physical gravity center of the three-dimensional image of the bone region Pi, wherein a denotes a constant, 0 < a < 0.2;

the extraction unit for image of descending aorta is connected to the extraction structure for gravity center of heart, the extraction structure for gravity center of spine and the lung tissue removal unit, and is configured for acquiring an image of descending aorta of each group of CT sequence images based on the gravity center of heart and the gravity center of spine;

the new image acquisition unit is connected to the extraction unit for image of descending aorta, the lung tissue removal unit, the slice data storage structure for aorta layer and the slice data storage structure for non-aorta layer, and is configured for removing the lung, descending aorta, spine and ribs from the CT sequence images, to acquire new images.

5. The system for acquiring image of aorta based on deep learning according to claim 4, **characterized in that**, the region delineation unit for descending aorta comprises: an average grayscale value acquisition module, a layered slice module and a binarization processing module;

the average grayscale value acquisition module is connected to the lung tissue removal unit and the grayscale histogram structure, and is configured for acquiring one or more pixel points PO within the first image with a grayscale value greater than the grayscale threshold for the descending aorta $Q_{descending}$, and calculating an average grayscale value $\overline{Q}_1$ of the one or more pixel points PO;

the layered slice module is connected to the average grayscale value acquisition module and the lung tissue removal unit, and is configured for layered slicing the first image starting from its bottom layer to obtain a first group of two-dimensional sliced images;

the binarization processing module is connected to the layered slice module and the grayscale histogram structure, and is configured for, based on

$$\begin{cases} Q_k < Q_{descending}, P(k) = 0 \\ Q_{descending} \leq Q_k \leq 2\overline{Q}_1, \ P(k) = 1 \\ Q_k > 2\overline{Q}_1, P(k) = 0 \end{cases},$$

binarizing the sliced image, removing impurity points from the first image to obtain a binarized image, wherein k is a positive integer, $Q_k$ denotes the grayscale value corresponding to the k-th pixel point PO, and P(k) denotes the pixel value corresponding to the k-th pixel point PO.

6. The system for acquiring image of aorta based on deep learning according to claim 5, **characterized in that**, the region delineation unit for descending aorta further comprises: an rough acquisition module and an accurate acquisition module;

the rough acquisition module is connected to the binarization processing module, and is configured for setting an radius threshold of a circle formed from the descending aorta to an edge of the heart to $r_{threshold}$, acquiring an approximate region of the spine and an approximate region of the descending aorta based on the distance between the descending aorta and the heart being less than the distance between the spine and the heart;

the accurate acquisition module is connected to the rough acquisition module, and is configured for removing one or more error pixel points based on the approximate region of the descending aorta, i.e., a circle corresponding to the descending aorta.

7. The system for acquiring image of aorta based on deep learning according to claim 6, **characterized in that**, the data extraction device comprises: a connected domain structure and a feature data acquisition structure;

the connected domain structure is connected to the new image acquisition unit and is configured for acquiring a plurality of binarized images of the CT sequence images to be processed from the new image acquisition unit;

the feature data acquisition structure is connected to the connected domain structure, and is configured for acquiring a connected domain of each binarized image successively starting from the top layer, as well as a proposed circle center $C_k$, an area $S_k$, a proposed circle radius $R_k$, and a distance $C_k$-$C_{(k-1)}$ between the circle centers of two adjacent layers, a distance $C_k$-$C_1$ from the circle center $C_k$ of each layer of slice to the circle center of the top layer Ci, and an area $M_k$ of all pixels whose pixel points are greater than 0 in a layer pixel and whose pixel points are equal to 0 in the previous layer pixel and a filtered area $H_k$ corresponding to the connected domain, wherein k denotes the k-th layer of slice, $k \geq 1$; i.e., the feature data.

8. The system for acquiring image of aorta based on deep learning according to claim 7, **characterized in that**, the feature data acquisition structure is provided with a data processing unit, as well as a circle center acquisition unit, an area acquisition unit and an radius acquisition unit, respectively, connected to the data processing unit;

the data processing unit is configured for detecting 3 layers of slice successively starting from the top layer by using the Hoff detection algorithm, and obtaining 1 circle center and 1 radius from each layer of slice, forming 3 circles respectively; removing points with larger deviations from 3 circle centers to obtain a seed point $P_i$ of the descending aorta; acquiring a connected domain $A_1$ of the layer where the seed point $P_i$ is located; acquiring a gravity center of the connected domain $A_1$ as the proposed circle center $C_i$, and acquiring the area $S_1$ of the connected domain $A_1$ and the proposed circle radius $R_i$; acquiring a connected domain $A_2$ of the layer where the seed point $P_i$ is located, by using the $C_i$ as a seed point; expanding the connected domain $A_1$ to obtain an expanded region $D_i$, removing a portion overlapping with the expanded region $D_i$ from the connected domain $A_2$ to obtain a connected domain $A_2'$; setting a volume threshold $V_{threshold}$ for the connected domain, if a volume $V_2$ of the connected domain $A_2'$ being less than $V_{threshold}$, removing one or more points that are too far from the circle center $C_1$ of the previous layer, acquiring the filtered area $H_k$, making the gravity center of the connected domain $A_2'$ as a proposed circle center $C_2$, acquiring an area $S_2$ of the connected domain $A_2$ and a proposed circle radius $R_2$; repeating the method of the connected domain $A_2$, acquiring a connected domain of each binarized image successively, as well as a proposed circle center $C_k$, an area $S_k$, a proposed circle radius $R_k$, and a distance $C_k$-$Q_{(k-1)}$ between the circle centers of two adjacent layers, a distance $C_k$-$C_1$ from the circle center $C_k$ of each layer of slice to the circle center of the top layer $C_i$ corresponding to the connected domain;
the circle center acquisition unit is configured for storing the proposed circle centers $C_1$, $C_2$ ... $C_k$ ...;
the area acquisition unit is configured for storing the areas $S_1$, $S_2$ ... $S_k$ ..., and the filtered areas $H_1$, $H_2$ ... $H_k$ ...;
the radius acquisition unit is configured for storing the proposed circle radii $R_1$, $R_2$ ... $R_k$ ... .

9. The system for acquiring image of aorta based on deep learning according to claim 8, **characterized in that**, the aorta acquisition device comprises: a gradient edge structure and an acquisition structure for image of aorta;

the gradient edge structure is connected to the deep learning device and is configured for expanding aorta data; multiplying the expanded aorta data with original CT sequence image data, and calculating a gradient of each pixel point to obtain gradient data; extracting a gradient edge based on the gradient data; subtracting the gradient edge from the expanded aorta data; the acquisition structure for image of aorta is connected to the new image acquisition unit and the gradient edge structure, and is configured for generating a list of seed points based on a proposed circle center; extracting a connected domain based on the list of seed points, to obtain an image of aorta.

| database device<br>100 | → | deep learning device<br>200 | | data extraction device<br>300 | → | aorta acquisition device<br>400 |

FIG.1

| CT storage device<br>500 |

| database device<br>100 | → | deep learning device<br>200 | | data extraction device<br>300 | → | aorta acquisition device<br>400 |

FIG.2

| CT storage device<br>500 | → | image processing<br>structure<br>110 | → | slice data storage<br>structure for aorta layer<br>120 | | slice data storage structure<br>for non-aorta layer<br>130 |

FIG.3

FIG.4

FIG.5

```
    ┌────────────────────┐                ┌────────────────────┐
    │     grayscale      │                │    lung tissue     │
    │  histogram unit    │                │   removal unit     │
    │       111          │                │       113          │
    └────────────────────┘                └────────────────────┘
              │                                     │
              ▼                                     ▼
┌────────────────────┐    ┌────────────────────┐    ┌────────────────────┐
│ average grayscale  │    │ layered slice      │    │ binarization       │
│ value acquisition  │───▶│ module             │───▶│ processing module  │
│ module   1621      │    │ 1622               │    │ 1623               │
└────────────────────┘    └────────────────────┘    └────────────────────┘
                                                               │
                                                               ▼
┌─────────────────────────┐              ┌─────────────────────────┐
│ ┌─────────────────────┐ │              │ ┌─────────────────────┐ │
│ │ seed point          │ │◀─────────────│ │ Hoff detection      │ │
│ │ acquisition element │ │◀───────────┐ │ │ element  1626       │ │
│ │      1627           │ │            └─│ └─────────────────────┘ │
│ └─────────────────────┘ │              │                         │
│ accurate acquisition    │              │ rough acquisition       │
│ module   1625           │              │ module   1624           │
└─────────────────────────┘              └─────────────────────────┘
```

FIG.6

```
┌────────────────────┐    ┌────────────────────┐
│ connected domain   │    │   new image        │
│ structure          │◀───│ acquisition unit   │
│    310             │    │     117            │
└────────────────────┘    └────────────────────┘
         │
         ▼
┌ ─ ─ ─ ─ ─ ─ ─ ─ ─ ─ ─ ─ ─ ─ ─ ─ ─ ─ ─ ─ ─ ─ ─ ─ ─ ─ ─ ─ ─ ─ ─ ─ ─ ┐
  ┌────────────────┐  ┌────────────────┐  ┌────────────────┐  ┌────────────────┐
│ │ data processing│  │ circle center  │  │    area        │  │   radius       │ │
  │ unit           │─▶│ acquisition    │  │ acquisition    │  │ acquisition    │
│ │   321          │  │ unit  322      │  │ unit  323      │  │ unit  324      │ │
  └────────────────┘  └────────────────┘  └────────────────┘  └────────────────┘
│        │                                        ▲                  ▲          │
         └────────────────────────────────────────┴──────────────────┘
└ ─ ─ ─ ─ ─ ─ ─ ─ ─ ─ ─ ─ ─ ─ ─ ─ ─ ─ ─ ─ ─ ─ ─ ─ ─ ─ ─ ─ ─ ─ ─ ─ ─ ┘

           feature data acquisition structure
                         320
```

FIG.7

| deep learning device 200 | → | gradient edge structure 410 | → | acquisition structure for image of aorta 420 | ← | CT storage device 500 |

FIG.8

## INTERNATIONAL SEARCH REPORT

| International application No. |
|---|
| **PCT/CN2020/132798** |

**A.    CLASSIFICATION OF SUBJECT MATTER**

G06T 7/00(2017.01)i

According to International Patent Classification (IPC) or to both national classification and IPC

**B.    FIELDS SEARCHED**

Minimum documentation searched (classification system followed by classification symbols)

G06T

Documentation searched other than minimum documentation to the extent that such documents are included in the fields searched

Electronic data base consulted during the international search (name of data base and, where practicable, search terms used)

WPI; EPODOC; CNPAT; CNKI; IEEE: 深度学习, 主动脉, 图像, 数据库, 切片, 提取, 序列, 三维, CT, deep, learning, aorta, image, database, slice, sequence, 3D

**C.    DOCUMENTS CONSIDERED TO BE RELEVANT**

| Category* | Citation of document, with indication, where appropriate, of the relevant passages | Relevant to claim No. |
|---|---|---|
| X | CN 107563983 A (SHANGHAI UNITED IMAGING HEALTHCARE CO., LTD.) 09 January 2018 (2018-01-09)<br>    description paragraphs [0058]-[0169] | 1-2 |
| X | CN 110264465 A (CENTRAL SOUTH UNIVERSITY OF FORESTRY AND TECHNOLOGY) 20 September 2019 (2019-09-20)<br>    description paragraphs [0050]-[0139] | 1-2 |
| Y | CN 107563983 A (SHANGHAI UNITED IMAGING HEALTHCARE CO., LTD.) 09 January 2018 (2018-01-09)<br>    description paragraphs [0058]-[0169] | 3-6 |
| Y | CN 111815588 A (SUZHOU RUNXIN MEDICAL INSTRUMENT CO., LTD.) 23 October 2020 (2020-10-23)<br>    description paragraphs [0005]-[0112] | 3-6 |
| Y | CN 111815583 A (SUZHOU RUNXIN MEDICAL INSTRUMENT CO., LTD.) 23 October 2020 (2020-10-23)<br>    description paragraphs [0006]-[0176] | 3-6 |

☑ Further documents are listed in the continuation of Box C.        ☑ See patent family annex.

| | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **09 March 2021** | **26 March 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

International application No.

**PCT/CN2020/132798**

**C.     DOCUMENTS CONSIDERED TO BE RELEVANT**

| Category* | Citation of document, with indication, where appropriate, of the relevant passages | Relevant to claim No. |
|---|---|---|
| Y | CN 111815589 A (SUZHOU RUNXIN MEDICAL INSTRUMENT CO., LTD.) 23 October 2020 (2020-10-23) <br> description, paragraphs [0006]-[0211] | 3-6 |
| Y | CN 111815585 A (SUZHOU RUNXIN MEDICAL INSTRUMENT CO., LTD.) 23 October 2020 (2020-10-23) <br> description paragraphs [0006]-[0228] | 3-6 |
| A | CN 111815587 A (SUZHOU RUNXIN MEDICAL INSTRUMENT CO., LTD.) 23 October 2020 (2020-10-23) <br> entire document | 1-9 |
| A | CN 111815586 A (SUZHOU RUNXIN MEDICAL INSTRUMENT CO., LTD.) 23 October 2020 (2020-10-23) <br> entire document | 1-9 |
| A | CN 111815584 A (SUZHOU RUNXIN MEDICAL INSTRUMENT CO., LTD.) 23 October 2020 (2020-10-23) <br> entire document | 1-9 |
| A | US 2017235915 A1 (SIEMENS HEALTHCARE GMBH) 17 August 2017 (2017-08-17) <br> entire document | 1-9 |

Form PCT/ISA/210 (second sheet) (January 2015)

## INTERNATIONAL SEARCH REPORT
### Information on patent family members

International application No.

**PCT/CN2020/132798**

| Patent document cited in search report | | | Publication date (day/month/year) | Patent family member(s) | | | Publication date (day/month/year) |
|---|---|---|---|---|---|---|---|
| CN | 107563983 | A | 09 January 2018 | None | | | |
| CN | 110264465 | A | 20 September 2019 | None | | | |
| CN | 111815588 | A | 23 October 2020 | None | | | |
| CN | 111815583 | A | 23 October 2020 | None | | | |
| CN | 111815589 | A | 23 October 2020 | None | | | |
| CN | 111815585 | A | 23 October 2020 | None | | | |
| CN | 111815587 | A | 23 October 2020 | None | | | |
| CN | 111815586 | A | 23 October 2020 | None | | | |
| CN | 111815584 | A | 23 October 2020 | None | | | |
| US | 2017235915 | A1 | 17 August 2017 | CN | 107092771 | A | 25 August 2017 |

Form PCT/ISA/210 (patent family annex) (January 2015)